# EUROPEAN PATENT SPECIFICATION

(11) **EP 2 480 155 B1**
(45) Date of publication and mention of the grant of the patent: **20.11.2013**
(21) Application number: 10766390.8
(22) Date of filing: 21.09.2010
(51) Int. Cl.: G06Q 10/00, A61B 19/00, A61B 19/02, B65F 1/14, G06F 19/00

(54) **PHARMACY WASTE IDENTIFICATION LABELING AND DISPOSAL SYSTEM AND RELATED METHOD OF USING**
SYSTEM ZUR IDENTIFIZIERUNG, MARKIERUNG UND ENTSORGUNG VON PHARMAABFALL UND ZUGEHÖRIGES VERWENDUNGSVERFAHREN
SYSTÈME D'ÉTIQUETAGE D'IDENTIFICATION ET DE MISE AU REBUT DE DÉCHETS PHARMACEUTIQUES ET PROCÉDÉ D'UTILISATION ASSOCIÉ

(30) Priority: 21.09.2009 US 244270 P
(43) Date of publication of application: 01.08.2012
(73) Proprietor: Avery Dennison Corporation, Pasadena, CA 91103 (US)
(72) Inventor: DEHLINGER, Anne, M., Pendleton, NY 14120 (US); BECKER, William, Lockport, NY 14094 (US)
(74) Representative: Müller-Boré & Partner Patentanwälte
(86) International application number: PCT/US2010/049563
(87) International publication number: WO 2011/035277

(56) References cited:
- US-A1- 2008 190 953
- US-A1- 2008 237 341
- US-A1- 2009 206 995

## Description

### Field of the Invention

The present invention is directed to the field of hazardous waste management, and more particularly to pharmacy or medical waste management and a system and method for identifying, labeling and arranging for the proper disposal of such waste.

### Background of the Invention

Pharmacy waste, e.g. mendicants, pharmaceuticals, etc., has been recognized as an emerging problem as such waste material has been identified in municipal water drinking systems. One investigative report recently found that pharmaceutical products were detected in the drinking water supply of 24 major metropolitan areas. That report further identified that there are no sewage treatment systems that have been adequately engineered to remove such pharmaceutical products and pharmacy wastes from the waste stream.

Government waste management regulations have been put in place, and the Environmental Protection Agency (EPA) has announced that a mandatory survey be completed in 2009 in some 3500 facilities, including hospitals, long-term care facilities, hospices and veterinary facilities. There is a particularly serious concern in hospitals and other care facilities, due to the general lack of in-house expertise relating to medical and pharmaceutical waste disposal which is preventing the care facility from reaching full compliance with waste management regulations.

In situations where a pharmacy staff may be aware of the correct disposal procedures and methods, in most if not many cases, such trained or experienced staff is simply not present when the caregiver or other staff member disposes of the residual drug or pharmaceutical waste. Communication of the information and establishment of procedures regarding the disposal of thousands of drugs is a challenge for hospitals and other care facilities, especially as there are exceptions to disposal conditions depending on the amount of the residual drug or pharmaceutical waste that is remaining in the container.

Failure to comply with the relevant waste procedures can subject the hospital or care facility with steep criminal and civil finds that could be as much as $37,500 per violation. This situation can of course be exacerbated when one considers the number of times or instances each day when pharmaceutical products and residual drugs are discarded. Pharmacists can also be held personally liable and face fines and imprisonment where proper procedures are not followed.

If a hospital, long term care facility, hospice or veterinary facility were to take a "better safe than sorry" attitude and simply dispose of all waste in the "hazardous waste" category or receptacle, the facility would then suffer from significant disposal costs associated with such procedures, e.g. heavy duty incineration, to dispose of such material thus, leading to a general need to increase the costs associated with providing health care to the general citizenry.

Another issue that must be addressed with the disposal and handling of pharmaceutical waste products arises out of HIPPA regulations which requires the maintenance of patient information in strict confidence and as such, medicines with patient information must not be thrown in the thrash.
US 2008/0190953 discloses a pharmaceutical waste management system and method including RFID tags.

### Brief Summary of the Invention

The embodiments of the present invention described below are not intended to be exhaustive or to limit the invention to the precise forms disclosed in the following detailed description. Rather, the embodiments are chosen and described so that others skilled in the art may appreciate and understand the principles and practices of the present invention.
The invention is defined by appended claims 1 and 7, preferred embodiments are described in the dependent claims.

One aspect of the invention is related to a radio-frequency identification (RFID) tag that includes an RFID transceiver configured to transmit and receive radio frequency (RF) signals. The RFID transceiver has an integrated circuit (IC) coupled to an antenna having a changeable parameter including impedance, gain and directionality that, in conjunction with the characteristics of the IC defines a first read range of the RFID tag. The RFID tag also includes a releasable coupler configured to be releasably engagable with the RFID transceiver including a color coded coupling material identifying the level or type of waste material. The releasable coupler is configured such that when the releasable coupler is releasably engaged with the RFID transceiver, the coupling material alters at least one of the impedance, gain and directionality of the antenna to define at least second read range of the RFID tag corresponding to the type of waste material to signal to a particular waste receptacle to unlock. The second read range is different than the first read range.

Yet another aspect of the invention is related to a method for altering a read range of an RFID tag that includes providing the RFID tag in an engaged state that has an RFID transceiver which can provide RF signals in response to interrogation signals. The RFID tag also includes a releasable coupler that is engaged with the RFID transceiver to define a first read range for the RFID tag. The method also includes transitioning the RFID tag to a different state with a second read range that is substantially different than the first read range by disengaging at least a portion of the releasable coupler from the RFID transceiver to provide a different read range.

In yet another embodiment of the present invention, an identifier having a substantially quadrate substrate is provided having a first face and a second face. The substrate has first through third portions separable from one another by first and second perforation lines. The first portion having printed indicia related to a pharmaceutical component, the second portion having patient indicia that includes at least one of human or machine readable indicia and a third portion having a series of colored areas representing a series of waste categories. The third portion first substrate having a releasable coupler extending over each of the series of colored areas and separable and divisible into individual sections corresponding to the waste categories. One of the first and second faces having an RFID circuit coupled to the releaseable coupler, the RFID circuit is encoded with product information defining a particular waste level of the pharmaceutical product with which it is associated.

In a further embodiment, the first perforation line runs generally parallel to an edge of the substrate and the second perforation line has a contour arranged to separate the patient information from the substrate. The second perforation line contour creates an enlarged second portion area.

In a still further embodiment, the releaseable coupler has first through fifth read ranges corresponding to each of the waste categories and is removably, adhesively attached over the colored areas and a plurality of perforations separating the releaseable coupler into first through fifth removable sections. The RFID circuit is disposed near an end edge of the releaseable coupler and is capacitively coupled to the releaseable coupler. The RFID circuit is programmed with product information as well as waste type information for subsequent reading by a disposal system.

Another embodiment of the present invention pharmaceutical waste disposal system includes a pharmaceutical container containing a pharmaceutical. A tag having a substrate with first and second surfaces, first and second longitudinally extending sides and first and second transversely extending edges. The substrate has first through third portions. A RFID transceiver is attached to the third portion and a releasable coupler is capacitively coupled to the RFID transceiver. The RFID transceiver is encoded with product and waste type information. The coupler has a plurality of separable sections for selectively altering a parameter of the RFID transceiver. The third portion is attached to the pharmaceutical container. A plurality of pharmaceutical waste containers, each of the containers having a reader to selectively respond to a specific parameter or encoded information of the RFID transceiver.

The present invention represents a complete, enabling solution that allows pharmacists to identify the proper way to dispose of drugs, and through the use of an automated system, the disposal situation is communicated to the administration and caregivers of the healthcare facility responsible for discarding the waste material. The system utilizes one or more identification means, including color, radio frequency identification (RFID), changeable characteristics and combinations of the foregoing. The system requires some level of comparative recognition in order for the proper disposal unit to be unlocked or utilized for the removal of the medical waste material.

In a still further exemplary method for using the waste management tag as defined herein, a waste management tag is provided with an RFID transceiver and the RFID transceiver has an integrated circuit, antenna and releasable coupler and a color coding scheme to properly identify medical waste material. The tag is attached to a pharmaceutical product and the product is dispensed in a health care facility. A series of waste receptacles are provided with each of the receptacles being provided with a RFID reader to respond to and unlock one of the waste receptacles when the appropriate tag has been interrogated. A portion of the releasable coupler is removed so as to effectively and permanently alter the parameters of the antenna and the waste is disposed and secured.

Other features and advantages of the present invention will become apparent to those skilled in the art from the following detailed description. It is to be understood, however, that the detailed description of the various embodiments and specific examples, while indicating preferred and other embodiments of the present invention, are given by way of illustration and not limitation. Many changes and modifications within the scope of the present invention may be made without departing from the spirit thereof, and the invention includes all such modifications.

### Brief Description of the Drawings

These, as well as other objects and advantages of this invention, will be more completely understood and appreciated by referring to the following more detailed description of the presently preferred exemplary embodiments of the invention in conjunction with the accompanying drawings, of which:

FIGURE 1 depicts a front view of a pharmaceutical waste disposal tag utilizing a releasable coupler;

FIGURE 2 shows a further view of a pharmaceutical waste disposal tag with a releasable coupler;

FIGURE 3 provides a view of a disposal tag showing the separable portions of the tag;

FIGURE 3A depicts a cross sectional view of the tag of the present invention showing the releasable coupler in adhesive connection with the tag;

FIGURE 4 illustrates a schematic of a system to dispose of pharmaceutical action; and

FIGURE 5 provides a block diagram for an exemplary method of using the waste management tag as described herein.

### Detailed Description of the Invention

The present invention is now illustrated in greater detail by way of the following detailed description which represents the best presently known mode of carrying out the invention. However, it should be understood that this description is not to be used to limit the present invention, but rather, is provided for the purpose of illustrating the general features of the invention.

Automatic identification is the broad term applying to a host of technologies that are used to help machines identify objects. Automatic identification is often coupled with automatic data capture. Therefore, companies wanting to identify items are able to capture information about the items, to store the captured information in a computer, and to selectively retrieve the information from the computer for a variety of useful purposes, all with minimal human labor.

One type of automatic identification technology is radio-frequency identification (RFID). RFID is a term that describes technologies that use radio waves to automatically identify objects. There are several conventional methods of identifying objects using RFID, the most common of which is to store a serial number or data that identifies a product (and other information, if desired) on a microchip that is attached to an antenna. The chip and the antenna together define an RFID transceiver. The antenna enables a remote reader (e.g., an RFID reader) that has a transceiver to communicate with the chip, and enables the chip to transmit identification information back to the reader when actuated to do so (e.g.., interrogated) by the reader. The RFID reader converts the radio waves returned from the RFID tag into a form that can then be utilized by a computer.

Radio Frequency Identification (RFID) tags are used in a wide range of application environments. A typical RFID tag can include an RFID integrated circuit chip and antenna that is mounted on a substrate. Engaging and disengaging certain structures to the RFID tag can change certain properties of the antenna (e.g., the impedance of the antenna relative to the RFID integrated circuit chip, the gain of the antenna, the directionality of the antenna, etc.) which can alter the read range of the RFID tag.

As used herein the term container includes but is not limited to unit does packages, syringes, ampules, IV bags, bottles, vials, boxes, bottles, ampoule, and the like.

The RFID transceiver 104 could be mounted, for example, on a substrate 106 (e.g., a facestock). The substrate 106 could be formed, for example, with paper or plastic, as is known in the art and suitable RFID inlays are available from Avery Dennison RFID Company of Clinton, SC. The RFID inlay may be incorporated in a tag or label or included on a container or molded into a container during manufacture of the container. Additionally, the RFID enabled device or tag 102 can include a releasable coupler 108. The releasable coupler 108 can be releasably engagable with the substrate 106 and/or the RFID transceiver 104, such as through the use of a pressure sensitive adhesive. When the releasable coupler 108 is engaged to the substrate 106 and/or the RFID transceiver 104 (hereinafter referred to as an "engaged state"), the releasable coupler 108 increases the read range of the RFID tag 102 to a maximum read range (e.g., about 8 meters). It is to be understood that the term "read range" refers to both the range at which the RFID transceiver 104 can coherently receive interrogation signals transmitted from an external source (e.g., an RFID reader), as well as the range at which the external system can coherently receive a returned signal propagated from the RFID transceiver 104. In some implementations, the releasable coupler 108 can be disengaged from the substrate 106 and/or the RFID transceiver 104 by an end-user to substantially reduce the read range of the RFID transceiver 104, for example to about 15 centimeters. In other implementations, the RFID transceiver 104 can be configured such that when the releasable coupler 108 is disengaged from the substrate 106 and/or the RFID transceiver 104 (hereinafter referred to as a "disengaged state"), the far field read range of the RFID tag 102 can be substantially eliminated (e.g., less than 300 centimeters).

As an example, the releasable coupler 108 can include a section of coupling material. The coupling material could be implemented, for example, as a conductive material, such as conductive ink. In such an implementation, the conductive ink could be applied, for example with a printer with a ribbon or an ink-jet printer or laser printers. Exemplary printers are available under the trademark MONARCH from Avery Dennison Corporation, Printer System Division of Miamisburg, OH. Alternatively, the coupling material could be formed as a thin section of conductive metal (e.g., gold, copper or aluminum). In other implementations, the coupling material could be a vapor phase deposited metal. The conductive material could be incorporated into a strip of material such as paper or plastic and then the strip can be adhesively applied to the substrate. The choice of the particular coupling material can be based, for example, on the method of manufacturing or the materials chosen for the substrate 106. The interaction of the coupling material with the RFID transceiver 104 is dependent on the design and construction for the particular end use desired; however, in general, for a simple antenna such as a half wave dipole, the coupling material modifies the impedance of the antenna and hence alters the matching between the antenna and the RFID chip. As one example, the coupling material can electrically couple antenna segments (e.g., discrete sections) together, thereby changing the impedance of the antenna relative to the RFID chip. Such a change in the impedance can increase the read range of the RFID device.

Moreover, when the releasable coupler 108 is disengaged (e.g., removed) from the substrate 106 and/or the RFID transceiver 104, the RFID device is transitioned to the disengaged state. In the disengaged state, the coupling material no longer engages one or more of the antenna segments. Accordingly, the antenna segments electrically coupled by the coupling material are electrically decoupled in the disengaged state. As discussed above, the interaction of the coupling material with the RFID transceiver 104 is dependent on the design and construction of the RFID tag 102; however, in general, for a simple antenna such as a half wave dipole, the disengagement of the coupling material modifies the impedance of the antenna and hence alters the matching between the antenna and the RFID chip. For instance, such a decoupling could be configured to change the impedance of the antenna relative to the RFID chip, thereby substantially reducing (or even eliminating) the read range of the RFID device 102.

In another example, the engaging and disengaging of the coupling material could modify a gain and/or directionality associated with the antenna. For instance, the RFID enabled device 102 could be configured such that when the RFID device or tag 102 is in the engaged state, the coupling material can alter a radiation pattern of the antenna. Alteration of the radiation pattern could, for example, increase the sensitivity of the RFID tag 102 in certain directions, thereby altering an RFID read range in a given configuration of an RFID reader system and the RFID tag 102. Conversely, in such a configuration, when the RFID tag 102 is transitioned to the disengaged state, the radiation pattern of the antenna can be altered again, for example, to decrease the sensitivity of the antenna in certain directions, thereby altering the read range in the given configuration of the RFID reader system and the RFID tag 102. Moreover, a combination of the impedance, the gain and the directionality of the antenna could be adjusted to achieve specific characteristics (e.g., fine tuning) for the RFID tag 102.

The releasable coupler 108 can be releasably engaged to the substrate 106 and/or the RFID chip in a multitude of configurations. As one example, the releasable coupler 108 could be secured to the substrate 106 through an adhesive material (e.g., a non-curing pressure sensitive adhesive). Alternatively, the releasable coupler 108 could be engaged to the substrate 106 with a mechanical locking mechanism that could, for instance, require a specially designed tool for disengagement. As yet another example, the releasable coupler 108 could be formed as an integrated unit with the substrate 106, such that the releasable coupler 108 can be disengaged by tearing via perforations, slits, score lines or the like, 110, one or more portions 111, 112, 113, 114 or all of the releasable coupler 108 away from the substrate 106.

It should be appreciated that for certain application environments, it may be desirable to substantially reduce (or even eliminate) the read range of the RFID tag 102. For example, in waste management applications, a reader system (that includes an RFID reader) can be positioned at in or near waste receptacles to selectively lock and unlock based on the signal received from the RFID tag 102. The reader system detects and reads RFID tags that pass in the vicinity of the reader system. When a particular type of waste material with an attached RFID tag 102 is presented (e.g., when the item is placed near the waste receptacle or presented for verification), it is often undesirable for the associated RFID tag 102 to be read by the reader system that would unlock more than one waste receptacle. However, it may be desirable that the read range of the RFID tag 102 is not eliminated entirely to facilitate, for example, selectively unlocking or disabling one of the waste receptacles. Once the receptacle has been unlocked, a portion of the releasable coupler is removed to thus change the parameters of the antenna so that it will no longer function as before when the entire releasable coupler was in place. In such a situation, a short read range (e.g., 15 centimeters) would only allow an RFID tag 102 that is in very close proximity with an RFID reader to be interrogated and thus, for a verification check, a reader could read the contents of the waste receptacle to confirm that the contents are approved types of waste.

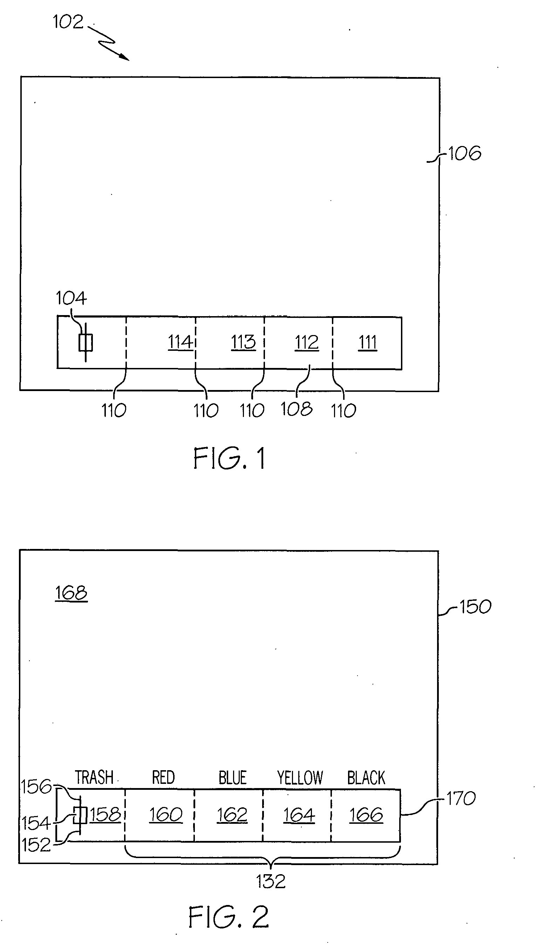

FIG. 2 illustrates a perspective view of an RFID tag 150 in accordance with an aspect of the invention. The RFID tag 150 is illustrated in an engaged state. In FIG. 2, different line patterns are employed to indicate different planes. As an example, the RFID tag 150 can have a substantially rectangular shape. The RFID tag 150 can include an RFID transceiver 152 that includes an RFID chip 154 for providing identification information to an associated antenna 156. The antenna 156 can transmit an RF signal that provides the identification information to an external system, such as an RFID reader in response to receiving an interrogation signal from the RFID reader. The antenna 156 can include a plurality of antenna segments 158-166 (e.g., discrete sections). It is to be understood that while the antenna segments 158-166 are illustrated as having a rectangular shape, other geometric configurations are possible. For example, the antenna segments could form a bar code, or be letters or other characters forming a word; the word, which becomes visible after the releasable coupler is disengaged, which could be indicative of the status of the RFID tag 150, such as "BLACK", "YELLOW", "BLUE", "RED" and "TRASH.". For example, when only one of the antenna segments 158 is electrically coupled to the RFID chip 154 when the RFID tag 150 is in a disengaged state and only the "TRASH" signal can be realized by a particular reader due to the encoding of the tag as well as the effective transmission range of the antenna.

The RFID tag 150 can include a releasable coupler 170 that can be releasably engagable with the substrate 168 and/or the RFID transceiver 152. The releasable coupler 170 includes a section of coupling material 172, which could be implemented as a conductive material, as discussed above. The releasable coupler 170 could be formed with a similar material as the substrate 168 (e.g., paper or plastic) or a different material. The releasable coupler 170, when engaged with the substrate 168, electrically couples the antenna segments 158-166 together (via the coupling material 172). Such electrical coupling of the antenna segments 158-166 increases a read range of the RFID tag 150 to a maximum (or near maximum) read range. The releasable coupler 170 could be disengaged from the substrate 168, for example by an end-user of the RFID tag 150. Once the releasable coupler 170 is disengaged, or portions of the coupler 170 the read range of the RFID tag 150 is substantially reduced, or reduced in increments by individually removing each of the segments.

In use, a health care professional would match a color associated with the type of medical waste and may then selectively remove one or more portions (158, 160, 162, 164, 166) of the coupler 170, such as by a line of weakness between each of the portions, so as to alter one of the predetermined parameters of the RFID transceiver 152, such as impedance, directionality or gain of the antenna 156. This will then create a unique reading arrangement when the tag is interrogated by waste receptacles once the material has been placed in the receptacles as discussed herein. US 20090206995 which is commonly assigned with the present invention, and entitled RFID Tag with a Releasable Coupler is hereby cited for a complete understanding of the present invention.

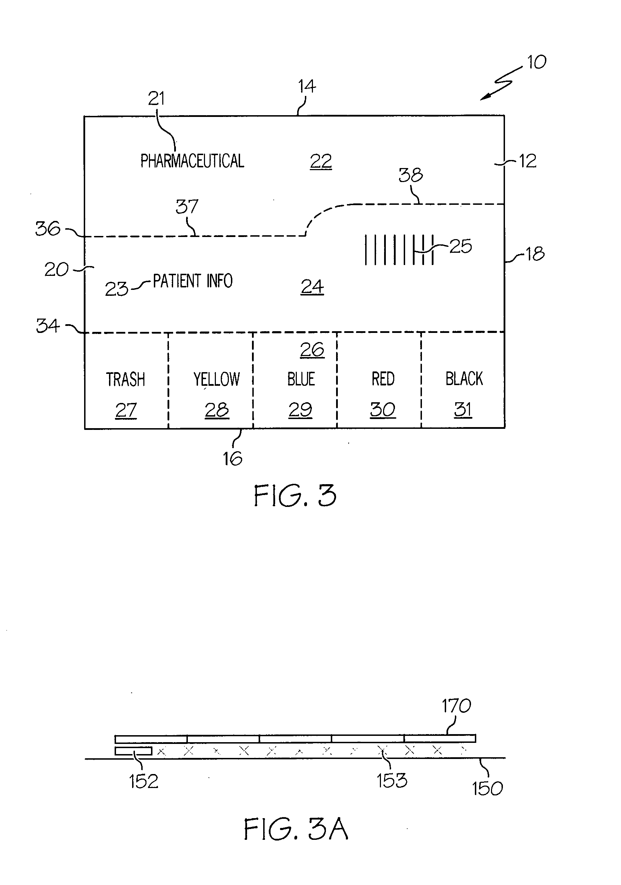

FIGURE 3 provides a view of the pharmaceutical waste identification tag 10 which has a first face 12 and a second face (not shown) opposite the first face. The tag 10 has first and second longitudinally extending side edges 14 and 16 and first and second transversely extending end edges 18 and 20. The first face 12 of the tag 10 is shown divided into three portions 22, 24 and 26. The first portion 22 is provided with indicia 21which may for example relate to the type of pharmaceutical product that is to be associated with the tag 10. The second portion 24 is provided with both human and machine readable indicia 23 and 25 respectively. The indicia 23 and 25 in second portion 24 relate to patient information. The third portion 26 on the first face 12 includes disposal information 27-31 which is color coded depending on the type of pharmaceutical waste that is created after the patient has been medicated. The second portion 24 would be adhesive free so as not to inadvertently catch on waste bins or in a shredding device thereby potentially exposing the patient information.

FIGURE 3 also shows first and second lines of weakness 34 and 36. The first line of weakness runs substantially parallel to the first and second longitudinal side edges 14 and 16 and the second line of weakness 36 has a first portion 37 that runs parallel to the first and second sides 14 and 16 and a second portion 38 that is used to contour the indicia 25 and to create a larger area so that the patient information may be removed to preserve confidentiality of the patient information.

FIGURE 3A shows a cross sectional of the tag of the presently described invention having a substrate 150, an RFID transceiver 152 and adhesive layer 153 on which the releasable coupler 170 is attached. The coupler 170 has a series of perforations allowing the coupler to be separated in to a number of segments.

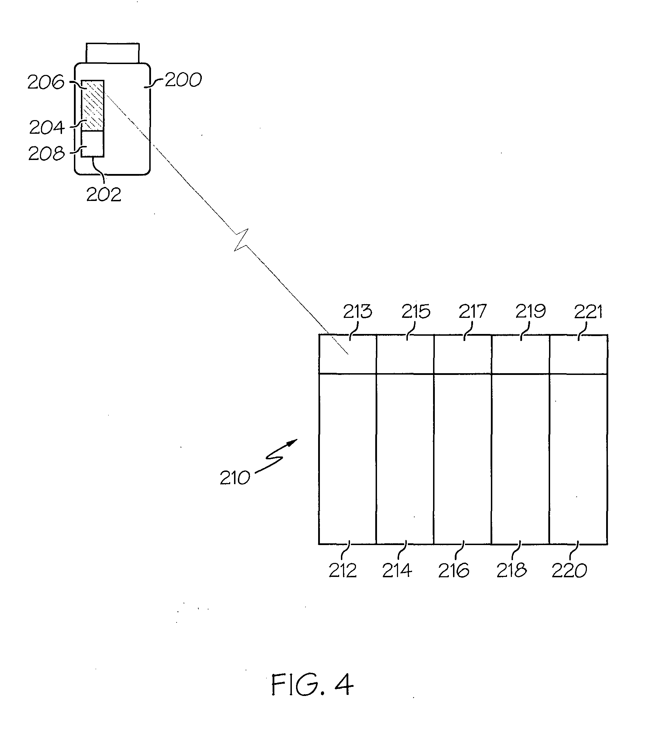

FIGURE 4 provides a schematic illustration of the pharmaceutical waste disposal system of the present invention. Pharmaceutical container 200 has the third portion 202 of the tag illustrated in FIGURE 3 above attached to the container 200 such as by a pressure sensitive adhesive. The third portion 202 is provided with an RFID transceiver 206 with a releasable coupler 204 in which a portion of the coupler 208 has been removed to impact the read range of the RFID transceiver 206 once the waste receptacle has read the encoded waste and or product information encoded in the tag and the waste receptacle has been opened. Thus, the tag cannot be used again as a far distance and can then only be read in at a short distance such as may be used to verify the contents of the waste receptacle.

A plurality of waste receptacles 212, 214, 216, 218 and 220 are shown in FIGURE 4 each with a RFID reader 213, 215, 217, 219, 221, associated with each of the receptacles. It should be understood, that while the receptacles are shown together or grouped, that the receptacles may be located apart from one another, in different secured areas so as to retain the integrity of the waste. The first reader 213 is shown interrogating the RFID transceiver 206 and when the signal is received the waste receptacle will be unlocked allowing the pharmaceutical waste to be discarded in the appropriate receptacle. The reader responds to the encoded information in the RFID transceiver and one or more parameters that are created by separating a portion of the releasable coupler from the transceiver. The parameters are selected from a group including impedance, gain or directionality of the antenna.

Subsequent to depositing the waste material in the receptacles, the receptacles can be scanned to read the product data associated with each chip to make sure that contents in the bin are correct and that hazardous material is not being discarded in a non-hazardous containing receptacle.

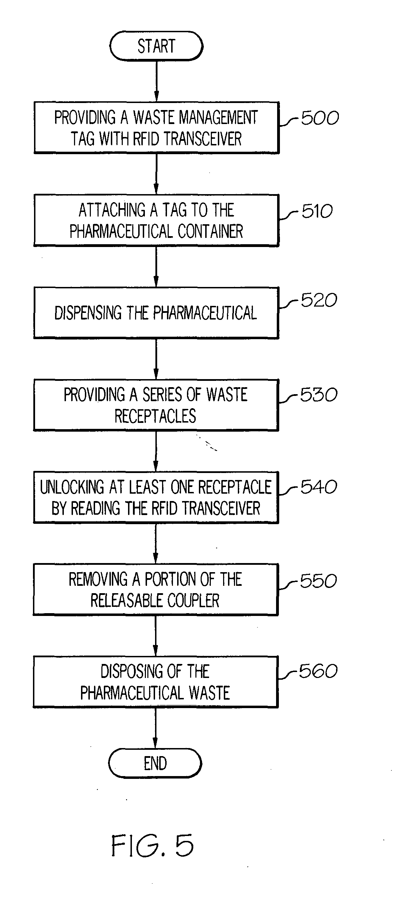

Reference is now directed to FIGURE 5 which shows a block diagram of an exemplary method for using the waste management tag as defined herein. In step 500 a waste management tag is provided with an RFID transceiver and a color coding scheme to properly identify medical waste material. Next, in step 510 the tag is attached to a pharmaceutical product and the product is dispensed at step 520. A series of waste receptacles are provided at step 530 which each of the receptacles being provided with a RFID reader to respond to and unlock one of the waste receptacles when the appropriate tag has been interrogated at step 540. Then at step 550 a portion of the releasable coupler is removed so as to effectively and permanently alter the parameters of the antenna and the waste is disposed of at step 560.

While RFID has been used as an exemplary embodiment, it should be understood that bar codes and two dimensional (2-D) bar codes could also be employed with optical scanning equipment and with or without RFID. Thus, the invention can be practiced with or without RFID such that at least one of RFID or a bar code can be used as the verification feature. In a situation where a bar code is employed, the bar code could be printed on the removable piece in lieu of a coupler and selectively removing portions of the piece would thus destroy portions of the bar code making the code unreadable to actuate another waste receptacle.

In addition, the system of the present invention can be used, upon reading a bar code or RFID device to initiate a signal to the health care professional asking if all of the medical or pharmaceutical material has been dispensed so that the container may be deposited in the TRASH receptacle thus avoiding the cost associated with having to dispose of hazardous waste material.

It will thus be seen according to the present invention a highly advantageous labeling and disposal system used in connection with pharmaceutical waste material has been provided. While the invention has been described in connection with what is presently considered to be the most practical and preferred embodiment, it will be apparent to those of ordinary skill in the art that the invention is not to be limited to the disclosed embodiment, and that many modifications and equivalent arrangements may be made thereof within the scope of the invention, which scope is to be accorded the broadest interpretation of the appended claims so as to encompass all equivalent structures and products.

The inventors hereby state their intent to rely on the Doctrine of Equivalents to determine and assess the reasonably fair scope of their invention as it pertains to any apparatus, system, method or article not materially departing from but outside the literal scope of the invention as set out in the following claims.

## Claims

1. A pharmaceutical waste disposal system comprising,
a pharmaceutical container (200) containing a pharmaceutical;
a tag (10), the tag comprising, a substrate (150) having first and second surfaces, first and second longitudinally extending sides (14, 16) and first and second transversely extending edges (18, 20), the substrate having first through third portions (22, 24, 26),
a RFID transceiver (152, 206) attached to the third portion (26), the RFID transceiver having an integrated circuit and an antenna with the RFID transceiver encoded with both product and waste type information; a releasable coupler (170) capacitively coupled to the RFID transceiver, the coupler having a plurality of separable sections (27-31) for selectively altering a parameter of the RFID transceiver; the third portion is attached to the pharmaceutical container; and
a plurality of pharmaceutical waste containers (212, 214, 216, 218, 220), each of the containers having a reader (213, 215, 217, 219, 221) to selectively respond to a specific parameter or encoded information of the RFID transceiver.

2. A pharmaceutical waste disposal system as recited in claim 1, wherein each of the separable sections (27-31) is color coded.

3. A pharmaceutical waste disposal system as recited in claim 1, wherein the releasable coupler (170) is removably attached to the tag.

4. A pharmaceutical waste disposal system as recited in claim 1, wherein the parameter of the RFID transceiver is selected from the group including impedance, gain or directionality of an antenna.

5. A pharmaceutical waste disposal system as recited in claim 1, wherein the separable sections of the releasable coupler are separable by lines of weak-ness.

6. A pharmaceutical waste disposal system as provided in claim 1, wherein the parameter of the antenna is selected from directionality, gain or impedance.

7. A method for disposing of pharmaceutical waste, comprising the steps of;
providing a pharmaceutical waste management tag (10), the tag having a RFID transceiver (152, 206), segmented, releasable coupler (170) and color coded indicia (27-31) corresponding to a particular pharmaceutical waste type;
attaching the tag to a pharmaceutical product (200);
dispensing the pharmaceutical product;
providing a series of waste receptacles (212, 214, 216, 218, 220), each receptacle having a RFID reader (213, 215, 217, 219, 221) to selectively unlock the receptacles;
reading the tag to unlock one of the receptacles;
removing a portion of the releasable coupler to permanently change an antenna parameter; and
disposing of the pharmaceutical waste product.

8. A method as provided in claim 7, wherein the parameter of the antenna includes one of directionality, gain or impedance.

9. A method as provided in claim 7, wherein the color scheme is provided on both the tag and the releasable coupler.

## Patentansprüche

1. Pharmaabfall-Entsorgungssystem, das umfasst:
einen Pharmabehälter (200), der ein pharmazeutisches Produkt enthält;
ein Etikett (10), wobei das Etikett ein Substrat (150) mit ersten und zweiten Oberflächen, erste und zweite sich längs erstreckende Seiten (14, 16) und erste und zweite sich quer erstreckende Ränder (18, 20) hat, wobei das Substrat erste bis dritte Abschnitte (22, 24, 26) hat,
einen RFID-Transceiver (152, 206), der an dem dritten Abschnitt (26) angebracht ist, wobei der RFID-Transceiver eine integrierte Schaltung und eine Antenne hat, wobei der RFID sowohl mit Informationen über das Produkt als auch den Abfalltyp codiert ist; einen lösbaren Koppler (170), der kapazitiv mit dem RFID-Transceiver gekoppelt ist, wobei der Koppler eine Vielzahl von trennbaren Abschnitten (27 - 31) hat, um einen Parameter des RFID-Transceivers selektiv zu ändern; wobei der dritte Abschnitt an dem Pharmabehälter angebracht ist; und
eine Vielzahl von Pharmaabfallbehältern (212, 214, 216, 218, 220), wobei jeder der Behälter ein Lesegerät (213, 215, 217, 219, 221) hat, um selektiv auf einen spezifischen Parameter oder codierte Informationen des RFID-Transceivers zu reagieren.

2. Pharmaabfall-Entsorgungssystem nach Anspruch 1, wobei jeder der trennbaren Abschnitte (27 - 31) farbcodiert ist.

3. Pharmaabfall-Entsorgungssystem nach Anspruch 1, wobei der lösbare Koppler (170) abnehmbar an dem Etikett angebracht ist.

4. Pharmaabfall-Entsorgungssystem nach Anspruch 1, wobei der Parameter des RFID-Transceivers aus der Gruppe ausgewählt wird, welche die Impedanz, die Verstärkung oder Richtwirkung einer Antenne umfasst.

5. Pharmaabfall-Entsorgungssystem nach Anspruch 1, wobei die trennbaren Abschnitte des lösbaren Kopplers durch Schwächungslinien trennbar sind.

6. Pharmaabfall-Entsorgungssystem nach Anspruch 1, wobei der Parameter der Antenne aus der Richtwirkung, der Verstärkung oder der Impedanz ausgewählt ist.

7. Verfahren zum Entsorgen von Pharmaabfall, das die folgenden Schritte umfasst:
Bereitstellen eines Verwaltungsetiketts (10) für Pharmaabfall, wobei das Etikett einen RFID-Transceiver (152, 206), einen segmentierten lösbaren Koppler (170) und farbcodierte Vermerke (27 - 31) hat, die einem bestimmten Typ von Pharmaabfällen entsprechen;
Anbringen des Etiketts an einem pharmazeutischen Produkt (200);
Abgeben des pharmazeutischen Produkts;
Bereitstellen einer Reihe von Pharmaabfallbehältern (212, 214, 216, 218, 220), wobei jeder der Behälter ein RFID-Lesegerät (213, 215, 217, 219, 221) hat, um die Behälter selektiv zu entriegeln;
Lesen des Etikettes, um einen der Behälter zu entriegeln;
Entfernen eines Abschnitts des lösbaren Kopplers, um einen Antennenparameter dauerhaft zu ändern; und
Entsorgen des Pharmaabfallprodukts.

8. Verfahren nach Anspruch 7, wobei der Parameter der Antenne die Richtwirkung, die Verstärkung oder die Impedanz umfasst.

9. Verfahren nach Anspruch 7, wobei das Farbschema sowohl auf dem Etikett als auch dem lösbaren Koppler bereitgestellt ist.

## Revendications

1. Système de mise au rebut de déchets pharmaceutiques, comprenant un récipient pharmaceutique (200) contenant un agent pharmaceutique ;
une étiquette (10), l'étiquette comprenant un substrat (150) ayant une première et une deuxième face, un premier et un deuxième côté s'étendant longitudinalement (14, 16), et un premier et un deuxième bord s'étendant transversalement (18, 20), le substrat ayant une première partie, une deuxième partie et une troisième partie (22, 24, 26),
un émetteur-récepteur RFID (152, 206) attaché à la troisième partie (26), l'émetteur-récepteur RFID présentant un circuit intégré et une antenne avec l'émetteur-récepteur RFID contenant à la fois des informations sur le type de produit et de déchet ; un coupleur amovible (170), couplé de manière capacitive à l'émetteur-récepteur RFID, le coupleur ayant une pluralité de sections séparables (27-31) pour modifier sélectivement un paramètre de l'émetteur-récepteur RFID ; la troisième partie est attachée au récipient pharmaceutique ; et
une pluralité de conteneurs pour déchets pharmaceutiques (212, 214, 216, 218, 220), chacun des conteneurs ayant un lecteur (213, 215, 217, 219, 221) pour répondre sélectivement à un paramètre spécifique ou une information enregistrée de l'émetteur-récepteur RFID.

2. Système de mise au rebut de déchets pharmaceutiques selon la revendication 1, où chacune des sections séparables (27-31) présente un code couleur.

3. Système de mise au rebut de déchets pharmaceutiques selon la revendication 1, où le coupleur amovible (170) est attaché de manière amovible à l'étiquette.

4. Système de mise au rebut de déchets pharmaceutiques selon la revendication 1, où le paramètre de l'émetteur-récepteur RFID est choisi parmi le groupe consistant en l'impédance, le gain ou la directivité d'une antenne.

5. Système de mise au rebut de déchets pharmaceutiques selon la revendication 1, où les sections séparables du coupleur amovible sont séparables grâce à des lignes de fragilisation.

6. Système de mise au rebut de déchets pharmaceutiques selon la revendication 1, où le paramètre de l'antenne est choisi parmi la directivité, le gain ou l'impédance.

7. Procédé de mise au rebut de déchets pharmaceutiques, comprenant les étapes de :
approvisionnement d'une étiquette de gestion des déchets pharmaceutiques (10), l'étiquette ayant un émetteur-récepteur RFID (152, 206), un coupleur amovible, segmenté (170) et des marquages à code couleur (27-31) correspondant à un type particulier de déchet pharmaceutique ;
fixation de l'étiquette à un produit pharmaceutique (200) ;
distribution du produit pharmaceutique ;
approvisionnement d'une série de réceptacles à déchets (212, 214, 216, 218, 220), chaque réceptacle ayant un lecteur RFID (213, 215, 217, 219, 221) pour ouvrir sélectivement les réceptacles ;
lecture de l'étiquette pour ouvrir l'un des réceptacles ;
élimination d'une partie du coupleur amovible pour changer de manière permanente un paramètre d'antenne ; et
mise au rebut du produit déchet pharmaceutique.

8. Procédé selon la revendication 7, où le paramètre de l'antenne comprend la directivité, le gain ou l'impédance.

9. Procédé selon la revendication 7, où le schéma de couleur est pourvu à la fois sur l'étiquette et sur le coupleur amovible.
